# EUROPÄISCHE PATENTSCHRIFT

(11) **EP 2 826 514 B1**
(45) Veröffentlichungstag und Bekanntmachung des Hinweises auf die Patenterteilung: **19.06.2019**
(21) Anmeldenummer: 12007197.2
(22) Anmeldetag: 17.10.2012
(51) Int. Cl.: A61M 25/00

(54) **Katheter-Set**
Catheter set
Set à cathéter

(30) Priorität: 21.10.2011 DE 202011107025 U
(43) Veröffentlichungstag der Anmeldung: 21.01.2015
(73) Patentinhaber: Medical Service GmbH, 75378 Bad Liebenzell (DE)
(72) Erfinder: Hiesch, Bernhard, 75328 Schömberg (DE); Passadore, Ricardo, 20037 Paderno Dugnano (MI) (IT)
(74) Vertreter: Grünecker Patent- und Rechtsanwälte PartG mbB

(56) Entgegenhaltungen:
- EP-A1- 1 897 579
- WO-A2-2006/085331
- GB-A- 2 033 231
- US-A- 5 454 798
- US-A1- 2003 060 807

## Beschreibung

Die vorliegende Erfindung betrifft ein Katheter-Set gemäß Oberbegriff von Anspruch 1.

Solche Katheter-Sets werden beispielsweise für den intermittierenden Katheterismus eingesetzt. Intermittierender Katheterismus ist die wiederholte Entleerung der Harnblase durch einen dünnen Einmalkatheter. Grundsätzlich sollte der Einmalkatheterismus der Harnblase aseptisch durchgeführt werden, d.h. unter Verwendung steriler Materialien, Desinfektion des Harnröhreneingangs, Verwendung eines sterilen Gleitmittels und dem sterilen Einführen des Katheters. Zur einfachen Durchführung des intermittierenden Katheterismus gibt es eine Reihe käuflicher Sets, die alle notwenigen Einzelteile enthalten. Da die Anwender oft in ihrer Bewegung eingeschränkt sind, ist bei solchen Sets insbesondere eine einfache Handhabung wichtig. Ein entsprechendes Katheter-Set ist zum Beispiel aus der GB 2033231 A bekannt, die ein Katheter-Set gemäß dem Oberbegriff des Anspruchs 1 offenbart.

So ist aus der EP 1 641 510 bereits ein Blasenkatheter-Set bekannt, das eine sterile Verpackung mit einem darin angeordneten Blasenkatheter umfasst. Auf dem Katheterschaft des Blasenkatheters ist eine Manschette angeordnet. Die Manschette ist entlang des Katheterschafts verschiebbar. Bei der Benutzung des Blasenkatheters wird die Verpackung geöffnet und der Blasenkatheter herausgenommen. Dabei kann der Anwender den Blasenkatheter an der Manschette greifen, so dass eine sterile Handhabung des Katheters möglich wird. Da der Katheter zur Benutzung allerdings aus der sterilen Verpackung herausgenommen wird, ist immer eine Kontaminationsgefahr gegeben.

Auch die US 5,454,798 zeigt ein Blasenkatheter-Set mit einem Blasenkatheter, der in einer sterilen Verpackung angeordnet ist. Die Verpackung ist an ihren Enden jeweils mit Siegelnähten verschlossen. Am vorderen Ende der Verpackung, d.h. an dem Ende, an dem der Katheter aus der Verpackung entnommen wird, ist eine Einführhilfe auf dem Katheter angeordnet. Das hintere Ende der Einführhilfe ist mittels der vorderen Siegelnaht an der Verpackung befestigt. Die Einführhilfe umgibt die Katheterspitze und bildet eine Ausschuböffnung für den Katheter aus. Die Verpackung ist über die vordere Siegelnaht hinaus verlängert und mittels einer dritten Siegelnaht verschlossen, so dass auch die Katheterspitze steril verpackt ist. Zwischen dem hinteren Ende der Einführhilfe, das in der Siegelnaht fixiert ist, und dem vorderen Ende der Einführhilfe, das die Katheterspitze umgibt, ist ein Faltenbalg angeordnet. Um den Katheter einzuführen greift der Benutzer die beiden Enden der Einführhilfe und zieht den Katheter mittels Greifen und Entlasten der beiden Enden und Ein- und Auffalten des Faltenbalges aus der Verpackung heraus. Bei der Einführhilfe handelt es sich also um eine relativ komplizierte Konstruktion.

Die US 6,578,709 B1 zeigt noch ein weiteres Blasenkatheterset mit einer sterilen Verpackung, in der ein Katheter und eine Benetzungsvorrichtung eingeschlossen sind. Die Benetzungsvorrichtung ist als Benetzungskammer ausgebildet, die an einem Ende eine Öffnung aufweist, an der Katheter zur Benutzung nach außen geschoben werden kann. Das Gehäuse der Benetzungskammer wird beim Einführen des Katheters als Greifhilfe verwendet. Beim Einführen des Katheters hält der Benutzer daher das Gehäuse mit einer Hand und greift mit den Fingern der anderen Hand durch die Verpackung das andere Ende des Katheters. Dann wird die Hand, die den Katheter durch die Verpackung hält nach vorne, zur Benetzungskammer bewegt und der Katheter durch die Öffnung in der Benetzungskammer nach außen geschoben. Die Benetzungskammer bildet also die Greifhilfe und die Ausschuböffnung für den Katheter aus.

Auch die US 4,230,115 zeigt ein Katheterset mit einer versiegelten Verpackung, in der ein Katheter, vorzugsweise ein Blasenkatheter, angeordnet ist. In der Verpackung ist eine Verjüngung ausgebildet, in der eine röhrenförmige Greifhilfe angeordnet ist. Die Spitze des Katheters ist in der Greifhilfe angeordnet. Durch die Verjüngung in der Verpackung werden in der Verpackung zwei Kammern ausgebildet. Eine erste Kammer, die zwischen einem ersten Ende der Verpackung und der Greifhilfe ausgebildet ist und die eine Sollbruchstelle aufweist, und eine zweite Kammer zwischen der Greifhilfe und dem zweiten Ende der Verpackung, in der der Katheter angeordnet ist. Zum Einführen des Katheters wird die Verpackung an der Sollbruchstelle geöffnet und in die erste Kammer ein Gleitmittel eingeführt. Dann wird der Penis in diese erste Kammer eingesteckt. Dadurch wird die Harnröhre automatisch mit der Bohrung der Greifhilfe ausgerichtet. Dann wird der Katheter durch die Greifhilfe in die Harnröhre eingeführt. Somit bildet die Greifhilfe die Ausschuböffnung für den Katheter aus.

Noch ein weiteres Blasenkatheterset ist in der US 6,053,905 gezeigt. Auch dieses Blasenkatheterset umfasst eine Verpackung, in der der Blasenkatheter angeordnet ist. An einem Ende der Verpackung ist eine Katheterausschuböffnung angeordnet. Vor dieser Katheterausschuböffnung befindet sich eine Gleitmittelkammer, durch die der Katheter bei der Benutzung geschoben und mit Gleitmittel benetzt wird. Am Ausgang der Gleitmittelkammer, d.h. der Katheterausschuböffnung, ist eine Greifhilfe angebracht. Zudem können in der Verpackung zwei parallele Siegelnähte vorgesehen sein, die eine Katheterführung ausbilden.

Auch die WO 03/008028 offenbart ein Katheterset mit einer Verpackung, in der ein Blasenkatheter angeordnet ist. In einer Abschlusssiegelnaht der Verpackung ist ein Bauteil befestigt, das eine Gleitmittelkammer, eine Greifhilfe und eine Ausschuböffnung ausbildet. Auch hier wird beschrieben, dass der Benutzer zum Einführen des Katheters das vordere Ende der Katheters mit Hilfe der Greifhilfe greift und das hintere Ende des Katheters durch die Wände der Verpackungen hindurch greift und den Katheter in Richtung der Ausschuböffnung der Verpackung bewegt.

Ein weiteres Katheter-Set ist in der US 2003/0060807 A1 beschrieben.

Es ist die Aufgabe der Erfindung, bereits bekannte Katheter-Sets weiter zu verbessern. Die Handhabung der Katheter-Sets soll weiter vereinfacht werden. Zudem ist eine einfache und kostengünstige Herstellung erwünscht.

Diese Aufgabe wird erfindungsgemäß durch ein Katheter-Set gemäß Anspruch 1 gelöst.

Insbesondere wenn der Katheter bzw. Katheterschaft mit Gleitmittel beschichtet ist, weist er eine sehr rutschige Oberfläche auf und ist daher nur schwer zu greifen. Durch die an der Verpackung fixierte Greifhilfe ist ein leichtes Greifen des Katheters möglich. Der Katheter kann nicht wegrutschen. Der Abstand zwischen der Greifhilfe und der Ausschuböffnung in der Verpackung erleichtert das Einführen des Katheters. Da die Greifhilfe an der Verpackung angeordnet ist und somit im Gebrauch in der Verpackung verbleibt, ist eine sterile Handhabung garantiert. Weiterhin ist vorgesehen, dass der Katheter an seinem der Ausschuböffnung abgewandten Ende einen Anschlag aufweist und der Innendurchmesser der Greifhilfe größer ist als der Außendurchmesser des Anschlags. Somit kann auch der Anschlag durch die Greifhilfe durchgezogen werden. Der Durchmesser der Ausschuböffnung ist kleiner als der Außendurchmesser des Anschlags. Der Katheter kann also nicht komplett aus der Verpackung gezogen werden. Das Katheterende wird in der Verpackung zurückgehalten und dichtet die Ausschuböffnung ab. Somit wird ein Auslaufen der Verpackung verhindert und gute Hygiene gewährleistet. Mittels der Greifhilfe kann der Anschlag gegen die Ausschuböffnung gedrückt werden, um die Dichtfunktion sicherzustellen.

In einer bevorzugten Ausgestaltung kann vorgesehen werden, dass die Greifhilfe als flexible Hülse ausgebildet ist. Bei der Hülse kann es sich um ein Schlauchstück, beispielsweise aus PVC, handeln. Dadurch wird eine sehr einfache Ausgestaltung der Greifhilfe möglich. Wird die flexible Hülse gegriffen, so kann der Katheterschaft durch Zudrücken eingeklemmt und einfach in der Verpackung nach vorne geschoben werden. Dadurch wird das Greifen des Katheterschaftes erleichtert. Bei der Hülse kann es sich um eine geschlossene oder geschlitzte Hülse handeln. Da der Katheterschaft über den gesamten Umfang von der Hülse umgeben ist, ist ein fester Griff möglich, der Katheterschaft kann nicht seitlich aus der Hülse herausrutschen.

Vorzugsweise kann ferner vorgesehen sein, dass die Ausschuböffnung in einem Verpackungsende ausgebildet ist. Dadurch ist eine sehr einfache Ausgestaltung der Ausschuböffnung möglich.

Vorteilhafterweise kann vorgesehen werden, dass die Ausschuböffnung durch eine Versteifung in der Verpackung ausgebildet ist. So kann beispielsweise ein Rohrstück an der Verpackung befestigt sein, das die Ausschuböffnung ausbildet. Dadurch werden zwei fixe Griffpunkte für den Katheterschaft ausgebildet, zum Einen die Greifhilfe, zum Anderen das die Ausschuböffnung ausbildende Rohrstück. Dadurch wird das Herausziehen des Katheterschafts aus der Verpackung und damit das Einführen des Katheter erleichtert.

Eine weitere Ausgestaltung kann vorsehen, dass der Abstand zwischen der Ausschuböffnung und der Greifhilfe mindestens ein Viertel der Länge des Katheterschaftes beträgt. Es hat sich gezeigt, dass bei diesem Abstand ein leichtes Einführen des Katheters möglich ist.

Ferner kann vorgesehen werden, dass die Greifhilfe durch mindestens eine Siegelnaht in der Verpackung fixiert ist. Dadurch ist eine sehr einfache Befestigung der Greifhilfe an der Verpackung möglich. Die Herstellung des Katheters wird vereinfacht. Um eine sichere Befestigung zu erreichen, kann vorzugsweise vorgesehen werden, dass die Greifhilfe von zwei einander gegenüberliegenden klammerförmigen Siegelnähten umgeben wird.

Vorteilhafterweise kann weiter vorgesehen werden, dass die Länge der Greifhilfe mindestens 10 % der Länge des Katheterschafts beträgt. Mit dieser Länge wird sichergestellt, dass der Benutzer die Greifhilfe einfach greifen kann.

Noch eine weitere Ausgestaltung kann vorsehen, dass der Innendurchmesser der Greifhilfe etwa das 1,5-fache bis 3-fache des Katheterdurchmessers beträgt. Der Innendurchmesser der Greifhilfe ist damit ausreichend groß, um ein einfaches Durchziehen des Katheterschaftes zu ermöglichen. Zudem ist der Innendurchmesser der Greifhilfe nicht zu groß, so dass eine gute Fixierung des Katheterschaftes in der Greifhilfe beim Zudrücken ermöglicht wird.

In einer weiteren bevorzugten Ausführungsform kann vorgesehen werden, dass die Verpackung als Urinauffangbehälter ausgebildet ist. Das Katheter-Set ist also ein geschlossenes System, so dass eine gute Hygiene ermöglicht wird.

Vorteilhafterweise kann vorgesehen werden, dass die Greifhilfe in dem Urinauffangbehälter angeordnet ist. Die Greifhilfe verbleibt also in der Verpackung, so dass eine Kontaminierung vermieden wird.

Vorzugsweise kann die Ausschuböffnung wiederverschließbar sein. Nach der Benutzung des Katheter-Sets wird der Katheter wieder zurück in die Verpackung geführt und die Ausschuböffnung wieder verschlossen, so dass gute Hygiene gewährleistet ist.

Es kann ferner vorgesehen werden, dass in der Verpackung ein Gleitmittel enthaltendes Sachet angeordnet ist. Bei diesem Gleitmittel handelt es sich um jede Art von Flüssigkeit, die die Reibung der Oberfläche des Katheterschafts verringert und das Einführen des Katheters erleichtert. Alle für die Benutzung des Katheters notwendigen Komponenten sind komplett in der Verpackung angeordnet, so dass eine einfache Handhabung ermöglicht wird. Durch Zerdrücken des Sachets wird das Gleitmittel in der Verpackung freigesetzt, der Katheter damit benetzt und somit benutzungsfertig gemacht.

Eine weitere Ausgestaltung kann vorsehen, dass der Katheter eine hydrophile Beschichtung aufweist. Damit wird die Bedienung des Katheters weiter erleichtert und die Gleitreibung beim Einführen minimiert.

In einer besonders bevorzugten Ausführungsform kann die Greifhilfe an einer Längsseite der Verpackung angeordnet sein. Dadurch wird die Aktivierung des Katheters mittels des Gleitmittels optimiert.

Im Folgenden wird die Erfindung anhand von Zeichnungen näher beschrieben. Es zeigen:
- Fig. 1: Katheter-Set,
- Fig. 2a: Greifhilfe des Katheter-Sets aus Fig. 1, und
- Fig. 2b: Schnitt durch die Greifhilfe aus Fig. 2 entlang Linie III-III.

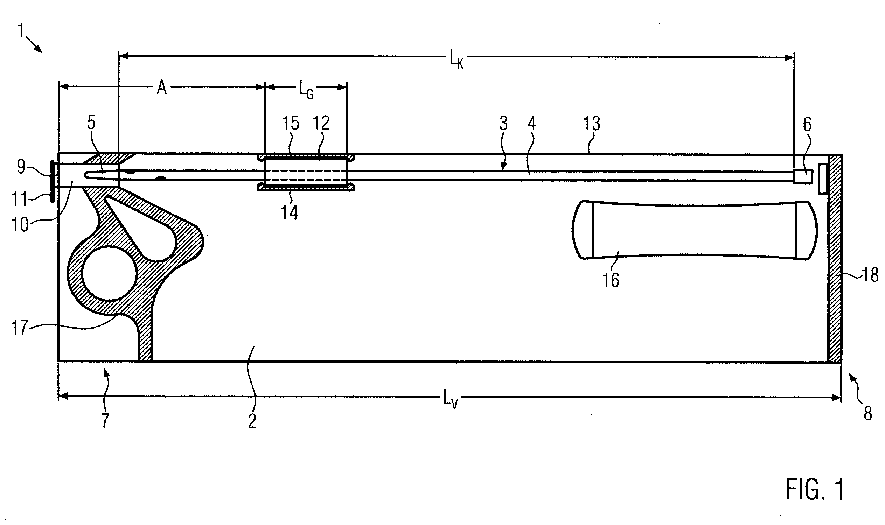

Fig. 1 zeigt ein Katheter-Set 1. Das Katheter-Set 1 umfasst eine Verpackung 2 und einen in der Verpackung 2 angeordneten Katheter 3. Der Katheter 3 wird mit der Verpackung 2 sterilisiert. Durch die Verpackung 2 kann die Sterilität des Katheters 3 bis zum Gebrauch sichergestellt werden.

Der Blasenkatheter 3 weist einen Katheterschaft 4, eine Katheterspitze 5 und an seinem der Katheterspitze 5 gegenüberliegenden Ende einen Anschlag 6 auf. Der Blasenkatheter 3 ist komplett in der Verpackung 2 angeordnet. Die Verpackung weist eine längsgestreckte Form auf. Es sind aber auch andere Formen denkbar. Als Material für die Verpackung 2 wird vorzugsweise eine PE-Folie eingesetzt. In dem dargestellten Beispiel wird die Verpackung 2 aus einem Folienschlauch hergestellt. Die Länge L_{V} der Verpackung 2 ist etwas größer als die Gesamtlänge des Katheters 3. Der Katheter 3 ist gestreckt in der Verpackung 2 angeordnet. An dem vorderen Ende 7 der Verpackung 2, d.h. an dem Ende, an dem die Katherspitze 5 angeordnet ist, und an dem hinteren Ende 8 der Verpackung 2, d.h. an dem Ende an dem der Anschlag 6 des Katheters 3 angeordnet ist, ist die Verpackung durch Siegelnähte 17, 18 verschlossen. In der vorderen Siegelnaht 17 ist eine Ausschuböffnung 9 ausgebildet. Dazu ist in der Siegelnaht 17 ein Rohrstück 10 befestigt, das die Ausschuböffnung 9 ausbildet. Das Rohrstück 10 weist einen Deckel 11 auf, so dass es wiederverschließbar ist. Die Katherspitze 5 ist in dem Rohrstück 10 und somit benachbart zu der Ausschuböffnung 9 angeordnet.

Im Inneren der Verpackung 2 befindet sich eine Greifhilfe 12. Die Greifhilfe 12 wird durch eine flexible Hülse, beispielsweise ein Schlauchstück, ausgebildet. Die Greifhilfe 12 ist an einer Längsseite 13 der Verpackung angeordnet. Mittels zweier einander gegenüberliegender Siegelnähte 14, 15 ist die Greifhilfe 12 an der Verpackung 2 befestigt. Die Siegelnähte 14, 15 sind klammerförmig ausgebildet und erstrecken sich entlang der Länge L_{G} der Greifhilfe 12. Am vorderen und am hinteren Ende der Greifhilfe 12 überragen die Siegelnähte 14, 15 die Greifhilfe 12 ein Stück nach innen. Der Katheter 3 ist durch die Greifhilfe 12 durchgeführt, so dass die Greifhilfe 12 den Katherschaft 4 umgibt. Der Innendurchmesser der Greifhilfe 12 ist größer als der Außendurchmesser des Katheterschafts 4 und der Außendurchmesser des Anschlags 6. Die Greifhilfe 2 ist also verschiebbar auf dem Katheterschaft 4 angeordnet, d.h. die Greifhilfe 12 kann in Bezug zu dem Katheterschaft 4 bewegt werden. Der Durchmesser der Ausschuböffnung 9 ist kleiner als der Außendurchmesser des Anschlags 6.

Die Greifhilfe 12 ist beabstandet zu der Ausschuböffnung 9 angeordnet. Dadurch ist zwischen der Ausschuböffnung 9 und der Greifhilfe 12 Verpackungsmaterial angeordnet. Das Verpackungsmaterial ist zusammenschiebbar, so dass die Greifhilfe 12 und die Ausschuböffnung 9 aufeinander zu bewegt werden können. Dies ist in Fig. 1 deutlich zu erkennen. Der Abstand A zwischen der Ausschuböffnung 9 und dem vorderen Ende der Greifhilfe 12 beträgt mindestens ein Viertel der Länge des Katheterschafts L_{K}. Vorzugsweise beträgt der Abstand A ein Viertel bis ein Drittel der Länge L_{K}.

In der Verpackung 2 ist ferner ein Sachet 16 angeordnet, das ein Gleitmittel enthält. Je nach Ausgestaltung des Katheters 3 bzw. des Katheterschafts 4 kann es sich bei dem Gleitmittel um eine Kochsalzlösung, Wasser oder ein Gleitgel bzw. jede andere Flüssigkeit handeln, die die Reibung des Katheters in der Harnröhre vermindert. Die Verpackung 2 ist als Urinauffangbehälter ausgebildet.

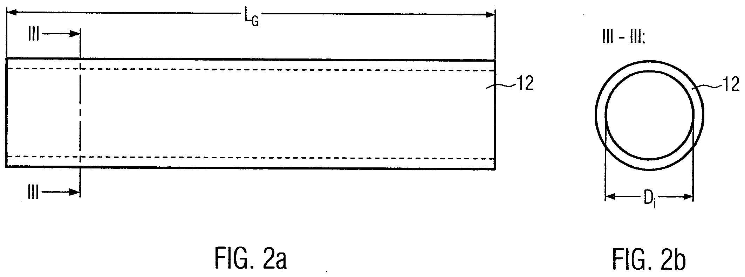

Fig. 2a zeigt die Greifhilfe 12 aus Fig. 1. Die Greifhilfe 12 ist hülsenförmig und wird durch ein weiches Schlauchstück ausgebildet. Vorzugsweise wird als Material für den Schlauch PVC verwendet. Die Länge der Greifhilfe L_{G} beträgt mindestens 10% der Länge des Katheterschafts L_{K}. Dadurch kann die Greifhilfe gut von einem Anwender gegriffen werden.

Fig. 2b zeigt einen Querschnitt durch die Greifhilfe 12 aus Fig. 1. Die Greifhilfe 12 hat die Form eines Hohlzylinders. Der Innendurchmesser Dᵢ der Greifhilfe 12 beträgt etwa das 1,5-bis 3-fache des Katheterdurchmessers. Die dargestellte Greifhilfe 12 ist über den gesamten Umfang geschlossen. Es kann auch vorgesehen werden, als Greifhilfe eine geschlitzte Hülse einzusetzen.

Zur Benutzung des Katheter-Sets 1 wird der Deckel 11 des Rohrstücks 10 und somit die Ausschuböffnung 9 geöffnet. Der Benutzer greift das Rohrstück 10 sowie die Greifhilfe 12. Durch Zusammendrücken der Greifhilfe 12 wird der Katheterschaft 4 fest gegriffen. Die Greifhilfe 12 wird dann Richtung Ausschuböffnung 9 bewegt und somit die Katheterspitze 5 durch das Rohrstück 10 aus der Verpackung 2 hinaus geschoben. Das zwischen der Ausschuböffnung 9 und der Greifhilfe 12 befindliche Verpackungsmaterial wird dabei zusammengeschoben. Die Katheterspitze 5 kann nun in die Harnröhre eingeführt werden. Nun wird das Rohrstück 10, das die Ausschuböffnung 9 definiert, zusammengedrückt und der Griff auf die Greifhilfe 12 gelöst. Die Greifhilfe 12 kann entlang des Katheterschafts 4 nach hinten geführt werden. Dadurch wird das zwischen der Greifhilfe 12 und der Ausschuböffnung 9 befindliche Verpackungsmaterial wieder gestreckt. Die Greifhilfe 12 wird nun wieder zusammengedrückt und Richtung Ausschuböffnung 9 bewegt, gleichzeitig wird der Griff auf das Rohrstück 10 gelockert, so dass der Katheterschaft 4 aus der Verpackung 2 hinaus geschoben wird. Durch Wiederholen dieses Vorgangs wird der Katheter 3 in der gewünschten Länge aus der Verpackung 2 hinausgeschoben. Vorzugsweise geschieht dies, bis der Anschlag 6 an dem Rohrstück 10 anliegt. Mittels der Greifhilfe 12 kann der Anschlag 6 so gegen das Rohrstück 10 gedrückt werden, dass eine dichte Verbindung entsteht. Nun kann Urin in die Verpackung 2 fließen, ohne dass Flüssigkeit austritt. Nach Abschluss des Vorgangs wird der Katheter 3 in die Verpackung 2 zurückgezogen und die Aufschuböffnung 9 mittels des Deckels 11 wieder verschlossen.

## Patentansprüche

1. Katheter-Set (1) mit einem Katheter (3), der einen Katheterschaft (4) aufweist, einer den Katheter (3) umgebenden Verpackung (2), die eine Ausschub-öffnung (9) für den Katheter (3) aufweist, und einer in der Verpackung (2) angeordneten Greifhilfe (12), die auf dem Katheterschaft (4) verschiebbar ist, wobei die Greifhilfe (12) derart beabstandet zu der Ausschuböffnung (9) an der Verpackung (2) befestigt ist, dass zwischen der Greifhilfe (12) und der Ausschuböffnung (9) zusammenschiebbares Verpackungsmaterial angeordnet ist, und wobei der Katheter (3) an seinem der Ausschuböffnung (9) abgewandten Ende einen Anschlag (6) aufweist, **dadurch gekennzeichnet, dass** der Innendurchmesser (D_{I}) der Greifhilfe derart größer ist als der Außendurchmesser des Anschlags (6), dass der Anschlag (6) durch die Greifhilfe (12) hindurchziehbar ist, und dass der Durchmesser der Ausschuböffnung (9) zum Zurückhalten des mit dem Anschlag (6) versehenen Katheterendes in der Verpackung (2) und zum Abdichten der Ausschuböffnung (9) kleiner ist als der Außendurchmesser des Anschlags (6).

2. Katheter-Set nach Anspruch 1, **dadurch gekennzeichnet, dass** die Greifhilfe (12) als flexible Hülse ausgebildet ist.

3. Katheter-Set nach Anspruch 1 oder 2, **dadurch gekennzeichnet, dass** die Ausschuböffnung (9) in einem Verpackungsende (7) ausgebildet ist.

4. Katheter-Set nach einem der Ansprüche 1 bis 3, **dadurch gekennzeichnet, dass** die Ausschuböffnung (9) durch eine Versteifung (10) in der Verpackung (2) ausgebildet wird.

5. Katheter-Set nach einem der Ansprüche 1 bis 4, **dadurch gekennzeichnet, dass** der Abstand (A) zwischen der Ausschuböffnung (9) und der Greifhilfe (12) mindestens ein Viertel der Länge (L_{K}) des Katheterschafts (4) beträgt.

6. Katheter-Set nach einem der Ansprüche 1 bis 5, **dadurch gekennzeichnet, dass** die Greifhilfe (12) durch mindestens eine Siegelnaht (14, 15) in der Verpackung (2) fixiert ist.

7. Katheter-Set nach einem der Ansprüche 1 bis 6, **dadurch gekennzeichnet, dass** die Länge (L_{G}) der Greifhilfe (12) mindestens 10% der Länge (L_{K}) des Katheterschafts (4) beträgt.

8. Katheter-Set nach einem der Ansprüche 1 bis 7, **dadurch gekennzeichnet, dass** der Innendurchmesser (D_{I}) der Greifhilfe (12) etwa das 1,5-fache bis 3-fache des Katheterdurchmessers beträgt.

9. Katheter-Set nach einem der Ansprüche 1 bis 8, **dadurch gekennzeichnet, dass** die Verpackung (2) als Urinauffangbehälter ausgebildet ist.

10. Katheter-Set nach Anspruch 9, **dadurch gekennzeichnet, dass** die Greifhilfe (12) in dem Urinauffangbehälter angeordnet ist.

11. Katheter-Set nach einem der Ansprüche 1 bis 10, **dadurch gekennzeichnet, dass** die Ausschuböffnung (9) wiederverschließbar ist.

12. Katheter-Set nach einem der Ansprüche 1 bis 11, **dadurch gekennzeichnet, dass** in der Verpackung (2) ein Gleitmittel enthaltendes Sachet (16) angeordnet ist.

13. Katheter-Set nach einem der Ansprüche 1 bis 12, **dadurch gekennzeichnet, dass** der Katheterschaft (4) eine hydrophile Beschichtung aufweist.

14. Katheter-Set nach einem der Ansprüche 1 bis 13, **dadurch gekennzeichnet, dass** die Greifhilfe (12) an einer Längsseite (13) der Verpackung (2) angeordnet ist.

## Claims

1. A catheter set (1) with a catheter (3), which comprises a catheter shaft (4), a packaging (2) surrounding the catheter (3), which comprises an opening (9) for extending the catheter (3), and a gripping aid (12) disposed in the packaging (2), which is displaceable on the catheter shaft (4), wherein the gripping aid (12) is fastened to the packaging (2) at a distance from the extension opening (9) in such a manner that a collapsible packaging material is disposed between the gripping aid (12) and the extension opening (9), and wherein the catheter (3) comprises a limit-stop (6) on its end facing away from the extension opening (9), **characterized in that**
the inner diameter (Di) of the gripping aid is greater than the outer diameter of the limit-stop (6), so that the limit-stop (6) can be pulled through the gripping aid (12) and that the diameter of the extension opening (9) is smaller than the outer diameter of the limit-stop (6) in order to retain the catheter end equipped with the limit-stop (6) inside the packaging (2) and to seal-off the extension opening (9).

2. The catheter set according to claim 1, **characterized in that** the gripping aid (12) is formed as a flexible sleeve.

3. The catheter set according to claim 1 or 2, **characterized in that** the extension opening (9) is formed in an end (7) of the packaging.

4. The catheter set according to one of the claims 1 to 3, **characterized in that** the extension opening (9) is formed by a reinforcement (10) in the packaging (2).

5. The catheter set according to one of the claims 1 to 4, **characterized in that** the distance (A) between the extension opening (9) and the gripping aid (12) amounts to at least a quarter of the length (L_{K}) of the catheter shaft (4).

6. The catheter set according to one of the claims 1 to 5, **characterized in that** the gripping aid (12) is fixed in the packaging (2) by at least one sealed seam (14, 15).

7. The catheter set according to one of the claims 1 to 6, **characterized in that** the length (L_{G}) of the gripping aid (12) amounts to at least 10% of the length (L_{K}) of the catheter shaft (4).

8. The catheter set according to one of the claims 1 to 7, **characterized in that** the inner diameter (Di) of the gripping aid (12) amounts to approximately 1.5 times to 3 times the diameter of the catheter.

9. The catheter set according to one of the claims 1 to 8, **characterized in that** the packaging (2) is designed as a urine collection container.

10. The catheter set according claim 9, **characterized in that** the gripping aid (12) is disposed in the urine collection container.

11. The catheter set according to one of the claims 1 to 10, **characterized in that** the extension opening (9) is reclosable.

12. The catheter set according to one of the claims 1 to 11, **characterized in that** a sachet (16) containing a lubricant is disposed in the packaging (2).

13. The catheter set according to one of the claims 1 to 12, **characterized in that** the catheter shaft (4) comprises a hydrophilic coating.

14. The catheter set according to one of the claims 1 to 13, **characterized in that** the gripping aid (12) is disposed on a longitudinal side (13) of the packaging (2).

## Revendications

1. Set de cathéter (1) avec un cathéter (3) qui comporte une tige de cathéter (4), un emballage (2) entourant le cathéter (3) et comportant une ouverture de sortie (9) pour le cathéter (3), et une aide à la préhension (12) disposée dans l'emballage (2) et qui est déplaçable sur la tige de cathéter (4), où l'aide à la préhension (12) est attachée à l'emballage (2) à distance de l'ouverture de sortie (9) de telle manière que du matériau d'emballage pliable est disposé entre l'aide à la préhension (12) et l'ouverture de sortie (9), et où le cathéter (3) comporte une butée (6) au niveau de son extrémité détournée de l'ouverture de sortie (9), **caractérisé en ce que** le diamètre intérieur (D_{I}) de l'aide à la préhension est plus grand que le diamètre extérieur de la butée (6), de sorte que la butée (6) peut être tirée à travers l'aide à la préhension (12) et que le diamètre de l'ouverture de sortie (9) est plus petit que le diamètre extérieur de la butée (6) afin de retenir l'extrémité du cathéter pourvue de la butée (6) dans l'emballage (2) et étanchéifier l'ouverture de sortie (9).

2. Set de cathéter selon la revendication 1, **caractérisé en ce que** l'aide à la préhension (12) prend la forme d'une douille flexible.

3. Set de cathéter selon la revendication 1 ou 2, **caractérisé en ce que** l'ouverture de sortie (9) est formée dans une extrémité de l'emballage (7).

4. Set de cathéter selon l'une des revendications 1 à 3, **caractérisé en ce que** l'ouverture de sortie (9) est formée par un renfort (10) dans l'emballage (2).

5. Set de cathéter selon l'une des revendications 1 à 4, **caractérisé en ce que** la distance (A) entre l'ouverture de sortie (9) et l'aide à la préhension (12) est égale à au moins un quart de la longueur (L_{K}) de la tige de cathéter (4).

6. Set de cathéter selon l'une des revendications 1 à 5, **caractérisé en ce que** l'aide à la préhension (12) est fixée dans l'emballage (2) par au moins un joint de scellage (14, 15).

7. Set de cathéter selon l'une des revendications 1 à 6, **caractérisé en ce que** la longueur (L_{G}) de l'aide à la préhension (12) est égale à au moins 10% de la longueur (L_{K}) de la tige de cathéter (4).

8. Set de cathéter selon l'une des revendications 1 à 7, **caractérisé en ce que** le diamètre intérieur (D_{I}) de l'aide à la préhension (12) est égal à 1,5 fois à 3 fois le diamètre du cathéter.

9. Set de cathéter selon l'une des revendications 1 à 8, **caractérisé en ce que** l'emballage (2) prend la forme d'une poche de recueil d'urine.

10. Set de cathéter selon la revendication 9, **caractérisé en ce que** l'aide à la préhension (12) est disposée dans la poche de recueil d'urine.

11. Set de cathéter selon l'une des revendications 1 à 10, **caractérisé en ce que** l'ouverture de sortie (9) est refermable.

12. Set de cathéter selon l'une des revendications 1 à 11, **caractérisé en ce qu'**un sachet (16) contenant un lubrifiant est disposé dans l'emballage (2).

13. Set de cathéter selon l'une des revendications 1 à 12, **caractérisé en ce que** la tige de cathéter (4) comporte un revêtement hydrophile.

14. Set de cathéter selon l'une des revendications 1 à 13, **caractérisé en ce que** l'aide à la préhension (12) est disposée sur un côté longitudinal (13) de l'emballage (2).
